(19) **Europäisches Patentamt**
**European Patent Office**
**Office européen des brevets**

(11) **EP 3 449 828 A1**

(12) **EUROPEAN PATENT APPLICATION**

(43) Date of publication:
**06.03.2019 Bulletin 2019/10**

(21) Application number: **18158483.0**

(22) Date of filing: **23.02.2018**

(51) Int Cl.:
*A61B 5/16* (2006.01)   *A61B 5/0472* (2006.01)
*A61B 5/00* (2006.01)   *G16H 50/20* (2018.01)
*A61B 5/04* (2006.01)   *A61B 5/024* (2006.01)
*A61B 5/0452* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **22.02.2018 PT 110584**
**28.08.2017 EP 17188217**

(71) Applicant: **INESC TEC - Instituto de Engenharia de Sistemas e Computadores, Tecnologia e Ciência**
**4200-465 Porto (PT)**

(72) Inventors:
- **da Silva Cunha, João Paulo Trigueiros**
  **4200-465 Porto (PT)**
- **Santos Paiva, Joana Isabel**
  **4520-022 Escapães, Santa Maria da Feira (PT)**

(74) Representative: **Patentree**
**Rua de Salazares 842**
**4149-002 Porto (PT)**

(54) **METHOD AND DEVICE FOR DETECTING STRESS USING BEAT-TO-BEAT ECG FEATURES**

(57)     Method and device for on-line detecting stress using individual heart beat ECG features of data acquired from a subject, comprising: obtaining a data sample of each heart beat individually from the acquired data; calculating the fiducial features RR, QR, RT, STinterval, STc, QTinterval, QTc, ST and QRS from each said data sample; classifying each data sample as stressed or not-stressed, using a pretrained classifier which was previously trained using the fiducial heart beat features RR, QR, RT, STinterval, STc, QTinterval, QTc, ST and QRS from previously acquired reference data samples from individual heart beats, determining stress as detected when at least one data sample is classified as stressed. Stress can be determined as detected when one data sample is classified as stressed over a time duration of: only one heart beat and the RR distance between said one heart beat and the previous heart beat.

EP 3 449 828 A1

## Description

### Technical field

The present disclosure relates to a method and device for detecting stress using beat-to-beat ECG features.

### Background

Recent years have seen a surge in the popularity and convenience of devices that collect physiological data. This has led to numerous efforts to use this data for a wide breadth of pertinent classification tasks such as the detection of cardiac arrhythmia, stress, sleep stages, drug use, and emotion [2]. Success in these classification tasks would have enormously broad and beneficial applications in many areas of public health including: preventing car accidents, increasing worker efficiency, mitigating health problems, monitoring drug use more effectively and improving Human-Computer Interaction [2].

Firefighting is one of the careers upon which stress has the largest negative impact. Firefighters are consistently exposed to stressful and fatiguing situations, giving them a higher risk of coronary diseases which account for a large percentage of deaths among these professionals.

Air Traffic Control (ATC) is also a very complex process [20]. It depends to a large degree on human abilities and it encompasses high levels of responsibility not only with regard to risking lives but also for the high economical costs of aeronautical activities. Much of the Air Traffic Controllers (ATCs) work complexity is characterized by unplanned tasks that require constantly re-plans in response to weather, traffic management initiatives, airport construction, maintenance activities, and other events [20].

The above reasons make both FFs and ATCs prime candidates for stress sensing experiments. In this way, simultaneously analyzing subject's perceived stress levels and physiological signals such as electrocardiogram (ECG) in both populations and other first responders, is the first step towards a general stress sensing solution, applicable to all contexts [2,4]. Since acute stress events induce physiological responses by our cardiovascular and neuroendocrine systems, ECG-derived features both in time and frequency domains have been widely used for stress monitoring and are highly correlated with subject's stress and arousal state changes [4]. Indeed, numerous authors have been exploring the use of ECG features in a human affect context. Most of these groups focus primarily on affect detection using Window-derived Heart Rate Variability (W-HRV) features [7]. However, this latter method has drawbacks. While the use of a window allows for a wide range of features to be used, including spectral features, these windows are usually 80 to 300 seconds which deteriorates the temporal resolution and increases the computational complexity of the detector in which such windows are used.

Methods for detecting stress should be evaluated not only accuracy, but also time resolution and computational rapidity of each method. Such metrics could be of high importance, since stressful events have been linked to abnormalities in cardiovascular functions (e.g. arrhythmias, cardiomyopathy, etc) in healthy and non-healthy persons, making prompt stress detection very desirable.

These facts are disclosed in order to illustrate the technical problem addressed by the present disclosure.

### General Description

In stress sensing, Window-derived Heart Rate Variability (W-HRV) methods are by far the most heavily used feature extraction methods. However, these W-HRV methods come with a variety of tradeoffs that motivate the development of alternative methods in stress sensing. We compare the presently disclosed method of using heart heat morphology (HBM) features for stress sensing to the traditional W-HRV method for feature extraction. In order to adequately evaluate these methods we conducted a Trier Social Stress Test (TSST) to elicit stress in a group of 13 firefighters and 11 Air Traffic Controllers (ATCs) while recording their ECG, actigraphy, and psychological self-assessment measures. We utilize the data from this experiment to analyze both feature extraction methods in terms of computational complexity, detection resolution performance, and event localization performance for each type of population separately (FFs and ATCs). Our results show that each method has an ideal niche for its use in stress sensing. W-HRV shows higher performance in stress event identification (7% higher in accuracy, 17% in precision and 22% in F1 score) but needs much more time to detect its presence than HBM (95% more - 16sec vs. 0.79sec). Thus the presently disclosed method, HBM, tends to be more effective in an online "fast" stress detection context where W-HRV shows to be more suitable for offline post processing scenarios. These results were reproducible on both occupational groups. Furthermore, the presently disclosed method, HBM, requires much less computational power than W-HRV.

It is disclosed a method for on-line detecting stress using individual heart beat ECG features of data acquired from a subject, comprising:

obtaining a data sample of each heart beat individually from the acquired data;

calculating the fiducial features RR, QR, RT, STinterval, STc, QTinterval, QTc, ST and QRS from each said data sample;

classifying each data sample as stressed or not-stressed, using a pretrained classifier which was previously trained using the fiducial heart beat features RR, QR, RT, STinterval, STc, QTinterval, QTc, ST and QRS from previously acquired reference data samples from individual heart beats,

determining stress as detected when at least one data sample is classified as stressed.

In an embodiment, stress is determined as detected when one data sample is classified as stressed over a time duration of

only one heart beat and the RR distance between said one heart beat and the previous heart beat.

In an embodiment, stress is determined as detected when 1-50, 1-20, 1-10, 1-5, 1-2, or just 1 data samples are classified as stressed.

In an embodiment, stress is determined as detected when a majority of data samples are classified as stressed over a predetermined duration of the acquired data.

In an embodiment, stress is determined as detected when a predetermined number of consecutive heart beats are classified as stressed, in particular 50, 20, 10, 5 or 2 consecutive heart beats are classified as stressed.

In an embodiment, the pretrained classifier is a Linear Support Vector Machine (SVM), a Kernel Support Vector Machine (K-SVM), a K-Nearest Neighbor (K-NN), or a Random Forest classifier.

In an embodiment, the pretrained classifier is a Random Forest Classifier.

In an embodiment, the detecting of stress comprises detecting stress under a predetermined time interval between a heart beat classified as stressed and the beginning of a stress interval of said subject.

It is also disclosed a non-transitory storage media including program instructions for implementing a method for on-line detecting stress using individual heart beat ECG features of data acquired from a subject, the program instructions including instructions executable to carry out any of the disclosed methods.

It is also disclosed a device for on-line detecting stress using individual heart beat ECG features of data acquired from a subject, comprising a data processor and data storage media configured for:

obtaining a data sample of each heart beat individually from the acquired data;

calculating the fiducial features RR, QR, RT, STinterval, STc, QTinterval, QTc, ST and QRS from each said data sample;

classifying each heart beat data sample as stressed or not-stressed, using a pretrained classifier which was previously trained using the fiducial features RR, QR, RT, ST, STc, QTc, ST and QRS from previously acquired reference data samples from individual heart beats,

determining stress as detected when at least one data sample is classified as stressed.

In an embodiment, the detecting of stress comprises detecting stress under a predetermined time interval between a heart beat classified as stressed and the beginning of a stress interval of said subject.

In an embodiment, the device is a wearable device.

**Brief Description of the Drawings**

The following figures provide preferred embodiments for illustrating the description and should not be seen as limiting the scope of invention.

**Figure 1:** Schematic representation of a diagram of an embodiment of the method. VAS - Visual Analogue Scales. TSST - Trier Social Stress Test, wherein 1 represents a start/end event, 2 represents an intermediate event, 3 represents visual scale, 4 represents self-reports.

**Figure 2:** Schematic representation of a portion of ECG from FF 2, with fiducial points Q, R, S and T; and points that contributed for extracting QT and ST intervals marked.

**Figure 3:** Schematic representation of a sketch of a heartbeat waveform illustrating six of the nine temporal intervals used in the classification task: QR segment; RT segment; ST interval; QT interval; ST segment and QRS segment.

**Figure 4:** Schematic representation of a summary of an embodiment of the classification method.

**Figure 5:** Schematic representation of a summary of an embodiment of the classification method used as a detection protocol.

## Detailed Description

The following pertains to methods of the disclosure, in particular to a description of a sample population dataset used in the present disclosure.

A dataset obtained from a population sample of 13 FFs and 11 ATCs with a high age variability (FFs: 3 female; age: $31 \pm 11$ years; ATCs: 3 female; age: $47 \pm 6$ years) was used in a study for demonstrating the advantages of the present disclosure. Data records from subjects with a history of cardiovascular disease and/or prescription cardiovascular-related drug use were not included.

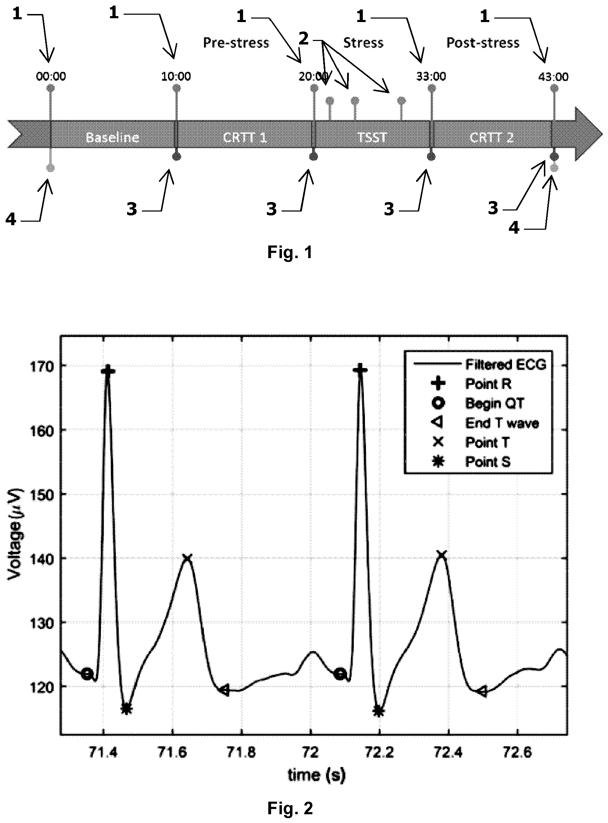

The following pertains to a description of the laboratory protocol of this study. The applied laboratory protocol (figure 1) was conducted in a previous study by our laboratory and proved to be a suitable protocol to induce acute stress in FFs [4]. ECG signals were continuously acquired throughout the duration of the experiment (approx. 1 hour) using the VitalJacket® [9] (VJ) at 500 Hz from a single lead. The VJ is a wearable bio-monitoring platform (in form of a t-shirt) able to collect ECG signals in a real-time manner, without affecting daily activities of users. It also contains a 3-axis Accelerometer system, allowing ECG signals correction for actigraphy profiles.

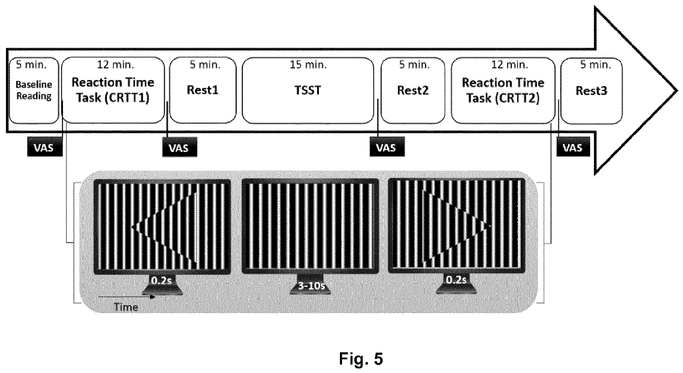

The laboratory protocol (see Fig. 5) performed by volunteers was composed of 3 main tasks during which they were comfortably sat in a chair. For evaluating the impact of stress in cognitive performance and after collecting an ECG baseline reading, a 2-choice reaction time task (CRTT1) [10] was conducted. This task required participants to respond to a stimulus, by clicking in a button, as fast as possible. The stimuli consisted in three different types of targets: (left screen) an arrowhead pointing to the left on top of a square both filled with a sinewave grating pattern; (right screen) the same stimulus, but with the arrowhead directed to the right; (middle screen) the grating square only (without an arrowhead) which remained in the center of the screen, between the appearance of each stimulus along the task - as illustrated in Figure 1. Following this, the Trier Social Stress Test (TSST) [8], a gold-standard psychological stress assessment procedure, was applied. After subjects were exposed to the stress condition, they performed again the simple CRTT (CRTT2) described above. Visual Analog Scales (VAS) [11] were used for stress psychological self assessment after each main task (after CRTT1, after TSST and after CRTT2). Participants were allowed to rest between each main event. All the ECG-based samples not belonging to the "TSST" event were labeled as "non-stress" and remaining ones as "stress".

The laboratory protocol performed by volunteers was composed of 3 main tasks during which they were comfortably sat in a chair. For evaluating the impact of stress in cognitive performance, a 2-choice reaction time task (CRTT) [10], was conducted. Following this, the Trier Social Stress Test (TSST) [8], a gold-standard psychological stress assessment procedure, was applied. After subjects were exposed to the stress condition, they performed again the simple CRTT (CRTT2) described above. Visual Analog Scales (VAS) were used for stress psychological self assessment after each main task (after CRTT1, after TSST and after CRTT2).

The following pertains to the ECG processing and features extraction. Since the primary method for feature extraction in the literature uses a W-HRV approach [7], where features are extracted from a fixed-length time interval with each sample representing a different shift of this interval, we compared the accuracy achieved in the proposed classification problem using W-HRV versus the disclosed HBM method both in FFs and ATCs. In HBM, each heartbeat waveform is treated as a separate sample.

In order to compare the two methods, we created a separate set of labelled samples for each method - see table I, for the presently used features in contrast with those used in prior art documents.

Table I: Enumeration of the features used in the classification task for each type.

| HeartBeat Morphology (HBM) Features [4], [12] | | Windowed Heart Rate Variability (W-HRV) Features [6], [7] |
| --- | --- | --- |
| 1. RR segment | | 1. Spectral power in [0-0.015] Hz |
| 2. $QR$ segment | | 2. Spectral power in [0.015-0.025] Hz band |
| 3. $RT$ segment | **Frequency Domain** | 3. Spectral power in [0.025-0.050] Hz band |
| 4. $ST$ interval | | 4. Spectral power in [0.050-0.120] Hz band |
| 5. $ST_C$ interval | | 5. Spectral power in [0.120-0.300] Hz band |
| 6. $QT$ interval | | 6. Spectral power in [0.300-0.400] Hz band |

(continued)

| HeartBeat Morphology (HBM) Features [4], [12] | | Windowed Heart Rate Variability (W-HRV) Features [6], [7] |
| --- | --- | --- |
| 7. $QT_C$ interval | | 7. AVNN (average of NN-intervals) |
| 8. *ST* segment | | 8. SDNN (standard deviation of *NN*-intervals) |
| 9. *QRS* segment | **Time Domain** | 9. rMSSD (square root of the mean squared difference of successive *NN* intervals) |
| | | 10. pNN50 (number of pairs of successive *NN* intervals that differ by more than 50 ms) |
| | | 11. RMS of the mean of the square of *NN* intervals |

The following pertains to presently disclosed method, HBM, features extraction. ECG heartbeats acquired during the different stages of the protocol were considered as samples with the temporal metrics extracted from each of these heartbeats as the features that characterize each respective sample of the dataset. ECG heartbeats (dataset samples) were therefore labelled as belonging to a "stressful" or "non-stressful" event according to the protocol stage in which they were acquired. Only the heartbeats that were collected in the TSST portion of the experiment were labelled as in the positive class, as per [4], with all others labelled as being in the negative class.

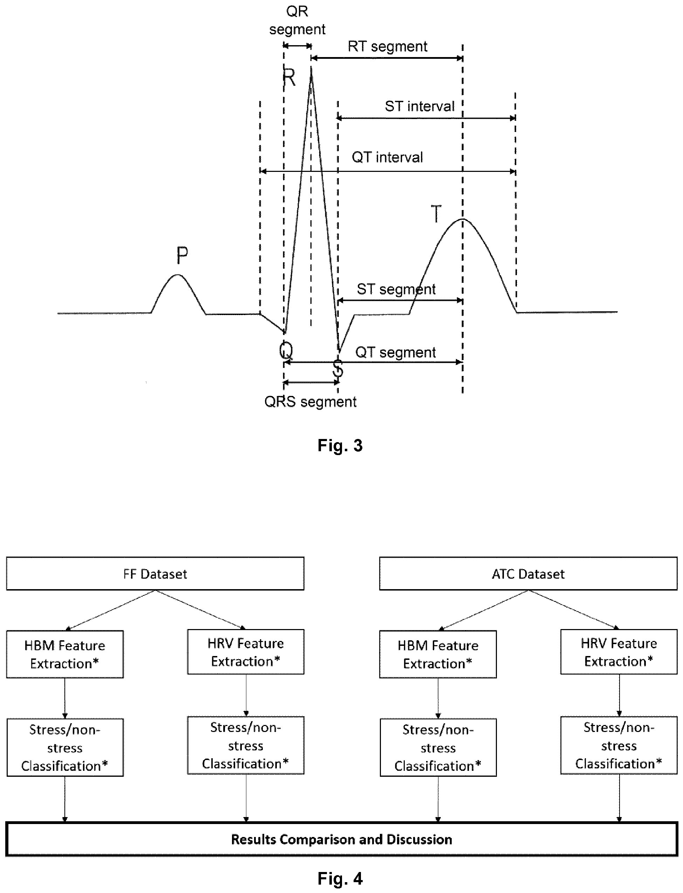

A set of nine features was extracted from each heartbeat waveform. The features used in this approach were based on temporal distances between fiducial points Q, R, S and T and were extracted using a ECG morphology-based patent pending [12, 21] processing scheme adopted in our previous study [4] - see figures 2 and 3.

R points were the first fiducials to be located, using the widely known Pan Tompkins algorithm [13]. Considering that existing literature shows that the best method for detecting ECG fiducial points is based on low order polynomial filtering, the remaining fiducials - Q, S and T - were located after applying a second order Butterworth lowpass filter with a cut off frequency of 10 Hz to the raw signal. Fiducial points were discovered based on previously established physiological time intervals [15]. The Q points were identified by computing the signal derivative considering a time window of 0.10 seconds before each R point. The last peak within this time window was marked as point Q for each heartbeat. Point S was located by applying a similar method, also based on signal derivatives. The first temporal mark, at which the derivative changed from negative to positive values, 0.05 seconds after the R point, was assigned as the point S. For locating the peak of the T wave, it was determined the last temporal index where the derivative of the signal changed from positive to negative values, within a time window of 0.05 to 0.40 seconds after each QRS complex, for each heartbeat.

QR, RT, ST and QRS segments were calculated as depicted in figure 3. RR intervals were defined as the interval between two consecutive R points. Contrary to the prior art, R is used as a temporal feature and in a less complex way, also less computationally heavy. The index of the beginning of QT was computed as the last point where the derivative changed from positive to negative in a time window of 0.03 seconds before each Q point. The end of the T wave was computed as the index corresponding to the last point at which the signal derivative changed from negative to positive values within 0.15 seconds after the T peak. $ST_C$ and $QT_C$ intervals were also included. These are the ST and QT intervals corrected for the interference of heart rate for each heartbeat, using the *Bazett Formula* [16]:

$$QT_C = \frac{QT}{\sqrt{RR}} \; , ST_C = \frac{ST}{\sqrt{RR}}$$

Through this process the QR, RT, ST, RR and QRS segments, as well as the ST, QT, $ST_C$ and $QT_C$ intervals were calculated for each heartbeat. The QT segment was also initially considered but revealed to be highly correlated to several other features, it was excluded in the classification task, leaving the remaining 9 features used in the HBM method - please see table I.

Noisy HBs were removed after computing all the nine temporal distance measures, by identifying the HBs which did not satisfy the following conditions [15]:

$$QR \leq 0.075 \, s \; and \; 0.200 < QT_C < 0.360 \, s$$

The following pertains to W-HRV features extraction. A fixed window of length 80 seconds was chosen through 10-fold cross validation over our training set with a generic Random Forest classifier. Each window was labeled as belonging to a stress event if the majority of the heartbeat waveforms contained in this window were labeled as belonging to a stress event.

In accordance with the literature [7], the Lomb-Scargle Periodogram was calculated on the RR-intervals determined with Pan Tompkins algorithm [13] and 6 spectral features were extracted based on the power in each of several bands, described in Table I [7]. In addition to spectral features, we also extracted 5 time-based HRV features (described in Table I) [7].

A total of 11 W-HRV features were therefore extracted from each window, contrasting with the 9 features in the HBM method (Table I).

The following pertains to the classification task. We trained and tested on the same person data in our protocol to evaluate the effectiveness of each method with regard to within subject (rather than between subject) event sensing - please see figure 4. This was done by dividing the samples in each subject into 5 equally sized, random groups and using a leave-one-out testing scheme. This was done 5 times such that in the end, every sample in each subject's time series had a score associated with it. These scores were then un-permuted so as to rearrange them back into the temporally sequential order in which they had been collected. This gave us a vector of scores for every sample in each subject's time series, in order. Using this score vector and the ground truth vector, we compared HBM to W-HRV in 5 different standard metrics: Accuracy, Precision, Recall, F1 Score, and the Area Under the Receiver Operating Characteristic curve (AUROC).

Several classifiers were compared for use in evaluating the effectiveness of HBM features versus W-HRV features. Among these models were: Linear Support Vector Machines (SVM), Kernel Support Vector Machines (K-SVM), K-NN (K-Nearest Neighbor) and Random Forest.

We used 5-fold cross validation grid search to find the best parameters for each subject for each model. Number of models in Random Forest was chosen by grid search from 2 to 70 in increments of 2. SVM C parameter grid search was from $10^{-4}$ to $10^4$ over factors of 10. The K-SVM sigma grid search was from $10^{-4}$ to $10^4$ over factors of 10. K-NN K grid search was from 1 to 20 by increments of 2.

After this was done, the model with the highest average cross validation fold F1-Score for each subject was used for the remainder of our experimentation and evaluation for that respective subject. In every case, the model with the highest performance on the validation set was a Random Forest Classifier, differing in the number of trees used depending on the subject and the method. The number of predictors sampled from on each tree split was the square root of the number of total predictors.

We compared HBM with W-HRV in three main areas: computational complexity, stress localization, and stress detection, which are discussed below.

The HBM features require only a single pass through the ECG signal to detect fiducial points and perform the elementary operations necessary to derive the associated features making the entire HBM method $O(n)$ in computation.

For each new shift of the W-HRV, 16 seconds are removed from the end of the old window and the next 16 seconds of the time series are added onto the front of the new window. The Lomb Periodogram is derived for the entire new window. The Lomb Periodogram is $O(n\log(n))$. The remaining HRV features are $O(n)$ making the entire W-HRV method $O(n\log(n)+n)$ $(O(n\log(n)))$.

This difference in computational complexity suggests different niches in stress event sensing where HBM features or W-HRV features shine. W-HRV features may not be as applicable to online sensing or wearable technology given the need for streamlined computation in these areas. Instead, W-HRV may be more suited for offline analysis of stress events.

Stress Localization is the process of determining the exact temporal bounds of a stress event. The nature of stress localization inherently gives equal importance to all samples within the stress event. With this in mind, in order to evaluate each method in this area, we used the metrics derived above. Average performance measures over all subjects (ATCs and FFs separately), for each method are shown in Table II.

Table II: Average test scores for each method and occupation.

| | | Accuracy | Precision | Recall | F1 Score | AUROC |
|---|---|---|---|---|---|---|
| **FF** | **HBM** | 0,837 | 0,703 | 0,595 | 0,628 | 0,845 |
| | **HRV** | 0,928 | 0,865 | 0,800 | 0,814 | 0,958 |
| | *difference* | *0,091* | *0,162* | *0,205* | *0,186* | *0,114* |
| **ATC** | **HBM** | 0,861 | 0,711 | 0,527 | 0,590 | 0,833 |
| | **HRV** | 0,906 | 0,889 | 0,817 | 0,845 | 0,956 |
| | *difference* | *0,045* | *0,178* | *0,289* | *0,255* | *0,123* |

HRV performance is better than HBM: difference of approx. 7% in accuracy, 17% in precision and 22% in F1 score (see table 2). The W-HRV method appears to be more effective for post processing the data offline when the goal is to determine the exact beginning, end, and duration of the stress event.

Unlike, stress localization, stress detection does not aim to determine exact bounds on the support of the event. Instead, it attempts to determine (as soon as possible) when the event begins, in an online fashion.

Let us define the Normalized Distance to Boundary (D), in percentage, as:

$$D = \frac{d}{L} \cdot 100$$

where d is the shortest distance between the predicted stress label and the beginning of the stress interval, and L is the length of the stress interval. In the following table, performance in terms of this measure is showed for each method and population in specific.

Table III: Normalized Distance to Boundary and Time Resolution.

|  |  | D (%) | Window Length (sec) | Time Resolution (sec) |
|---|---|---|---|---|
| **FF** | **HBM** | 1,55 | 0,79 * | 0,79 * |
|  | **HRV** | 2,03 | 80,00 | 16,00 ** |
|  | *difference* | 0,48 | 79.21 (99%) | 15,21 (95%) |
| **ATC** | **HBM** | 5,32 | 0.76 * | 0.76 * |
|  | **HRV** | 3,88 | 80,00 | 16,00 ** |
|  | *difference* | -1,44 | 79,24 (99%) | 15,24 (95%) |

\* average length of heart beat
\*\* window overlap of 80%

The following can be ascertained then:

- HRV and HBM have similar performance in detecting stress boundary (distance to stress boundary, metric D in table III).
- Needed window length to detect stress boundary is 98% smaller for HBM (see table III).
- Stress detection time resolution is 90% smaller for HBM (see table III).
- HRV is more accurate and precise but needs large windows and has low time resolution whereas HBM is less accurate and precise, but detects stress with a much larger time resolution, needing a very small window - only one heartbeat and its RR distance to the previous R peak, on average.
- If we are looking for a fast, cause/effect stress detector, HBM has much better characteristics than HRV. Nevertheless, if we want a precise and accurate stress detector and can sacrifice time resolution, HRV is a better method than HBM.

It is evident from this disclosure that W-HRV and HBM have specific applications in stress event sensing. W-HRV methods have slightly higher "accuracy" (F1 Score), yet they require more computation and do not achieve very good detection results.

In comparison, HBM methods require far less computation ((O(n)) computational complexity) and show excellent results in the area of detection. This makes the HBM method a excellent candidate for use in online processing and detection, while W-HRV methods are possibly more suitable for offline post-processing of the time series data. Therefore, each application should weigh the benefits of accurate detection with early detection. To provide a fair comparison, we used a roughly equivalent number of features for both.

The following references are incorporated by reference in their entirety.

[2] J. Cunha, "PHealth and wearable technologies: A permanent challenge," Stud. Health Technol. Inform, vol. 177, pp. 185-195, 2012.

[4] J. Paiva, S. Rodrigues, and J. Cunha, "Changes in ST, QT and RR ECG intervals during acute stress in firefighters: A pilot study," in Engineering in Medicine and Biology Society (EMBC), 2016 IEEE 38th Annual International Conference of the. IEEE, 2016, pp. 3378-3381.

[7] A. Voss, R. Schroeder, A. Heitmann, A. Peters, and S. Perz, "Shortterm heart rate variability - influence of gender and age in healthy subjects," PloS one, vol. 10, no. 3, p. e0118308, 2015.

[8] M. Birkett, "The Trier Social Stress Test protocol for inducing psychological stress," Journal of visualized experiments: JoVE, vol. 19, no. 56, 2011.

[9] J. Cunha, B. Cunha, A. Pereira et al., "Vital-jacket: A wearable wireless vital signs monitor for patients' mobility in cardiology and sports," in Pervasive Computing Technologies for Healthcare, 2010 4th International Conference on. IEEE, 2010, pp. 1-2.

[10] J. Paiva, "Predicting lapses in attention: a study of brain oscillations, neural synchrony and eye measures," MSc Thesis, University of Coimbra, pp. 33-36, 2014.

[12] J. Cunha and J. Paiva, "Biometric Method and Device for Identifying a Person Through an Electrocardiogram (ECG) Waveform - ref. PT109357," 2016, PT109357.

[13] J. Pan and W. Tompkins, "A real-time QRS detection algorithm," Biomedical Engineering, IEEE Transactions on, vol. 32, no. 3, pp. 230-236, 1985.

[15] D. Clifford, "ECG statistics, noise, artifacts, and missing data," Advanced Methods and Tools for ECG Data Analysis, vol. 6, pp. 55-99, 2006.

[16] H. Bazett, "An analysis of the time-relations of electrocardiograms," Heart, vol. 7, pp. 353-370, 1920.

[20] Koros A., D.R.P.S., Panjwani G., Ingurgio V., D'Arcy J.F. Complexity in air traffic control towers: A field study part 1. Complexity factors; Virginia 2003; pp 1-125.

[21] Paiva JS, Dias D, Cunha JPS (2017) Beat-ID: Towards a computationally low-cost single heartbeat biometric identity check system based on electrocardiogram wave morphology. PLOS ONE 12(7): e0180942. https://doi.org/10.1371/journal.pone.0180942.

The term "comprising" whenever used in this document is intended to indicate the presence of stated features, integers, steps, components, but not to preclude the presence or addition of one or more other features, integers, steps, components or groups thereof.

Flow diagrams of particular embodiments of the presently disclosed methods are depicted in figures. The flow diagrams do not depict any particular means, rather the flow diagrams illustrate the functional information one of ordinary skill in the art requires to perform said methods required in accordance with the present disclosure.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

Furthermore, it is to be understood that the invention encompasses all variations, combinations, and permutations in which one or more limitations, elements, clauses, descriptive terms, etc., from one or more of the claims or from relevant portions of the description is introduced into another claim. For example, any claim that is dependent on another claim can be modified to include one or more limitations found in any other claim that is dependent on the same base claim.

The disclosure should not be seen in any way restricted to the embodiments described and a person with ordinary skill in the art will foresee many possibilities to modifications thereof. The above described embodiments are combinable. The following claims further set out particular embodiments of the disclosure.

**Claims**

1. Method for on-line detecting stress using individual heart beat ECG features of data acquired from an individual subject, comprising:

   obtaining a data sample of each heart beat individually from the acquired data;
   calculating the fiducial features RR, QR, RT, STinterval, STc, QTinterval, QTc, ST and QRS from each said data sample;
   classifying each data sample as stressed or not-stressed, using a pretrained classifier which was previously trained using the fiducial heart beat features RR, QR, RT, STinterval, STc, QTinterval, QTc, ST and QRS from previously acquired reference data samples from individual heart beats of said individual subject;
   determining stress as detected when at least one data sample is classified as stressed.

2. Method according to claim 1 wherein stress is determined beat-to-beat and determined as detected when one data sample is classified as stressed over a time duration of
only one heart beat and the RR distance between said one heart beat and the previous heart beat.

3. Method according to any of the previous claims wherein the used fiducial features are only the fiducial features: RR, QR, RT, STinterval, STc, QTinterval, QTc, ST and QRS.

4. Method according to any of the previous claims wherein stress is determined as detected when 1-50, 1-20, 1-10, 1-5, 1-2, or only 1 data samples are classified as stressed.

**5.** Method according to any of the previous claims, wherein stress is determined as detected when a majority of data samples are classified as stressed over a predetermined duration of the acquired data.

**6.** Method according to any of the previous claims, wherein stress is determined as detected when a predetermined number of consecutive heart beats are classified as stressed, in particular 50, 20, 10, 5 or 2 consecutive heart beats are classified as stressed.

**7.** Method according to any of the previous claims, wherein the pretrained classifier is a Linear Support Vector Machine (SVM), a Kernel Support Vector Machine (K-SVM), a K-Nearest Neighbor (K-NN), or a Random Forest classifier, in particular the pretrained classifier is a Random Forest Classifier.

**8.** Method according to any of the previous claims, wherein the detecting of stress comprises detecting stress under a predetermined time interval between the beginning of a stress interval of said subject and a heart beat classified as stressed.

**9.** Non-transitory storage media including program instructions for implementing a method for on-line detecting stress using individual heart beat ECG features of data acquired from a subject, the program instructions including instructions executable to carry out the method of any of the claims 1-8.

**10.** Device for on-line detecting stress using individual heart beat ECG features of data acquired from an individual subject, comprising a data processor and data storage media configured for:

> obtaining a data sample of each heart beat individually from the acquired data;
> calculating the fiducial features RR, QR, RT, STinterval, STc, QTinterval, QTc, ST and QRS from each said data sample;
> classifying each heart beat data sample as stressed or not-stressed, using a pretrained classifier which was previously trained using the fiducial features RR, QR, RT, ST, STc, QTc, ST and QRS from previously acquired reference data samples from individual heart beats of said individual subject;
> determining stress as detected when at least one data sample is classified as stressed.

**11.** Device according to claim 10 wherein stress is determined beat-to-beat and determined as detected when one data sample is classified as stressed over a time duration of
only one heart beat and the RR distance between said one heart beat and the previous heart beat.

**12.** Device according to any of the claims 10-11 wherein the used fiducial features are only the fiducial features: RR, QR, RT, STinterval, STc, QTinterval, QTc, ST and QRS.

**13.** Device according to any of the claims 10-12, wherein the pretrained classifier is a Linear Support Vector Machine (SVM), a Kernel Support Vector Machine (K-SVM), a K-Nearest Neighbor (K-NN), or a Random Forest classifier, in particular the pretrained classifier is a Random Forest Classifier.

**14.** Device according to any of the previous claims 10-13, wherein the detecting of stress comprises detecting stress under a predetermined time interval between a heart beat classified as stressed and the beginning of a stress interval of said subject.

**15.** Device according to any of the claims 10-14, wherein the device is a wearable device.

EP 3 449 828 A1

Fig. 1

Fig. 2

Fig. 3

Fig. 4

Fig. 5

Europäisches
Patentamt

European
Patent Office

Office européen
des brevets

**EUROPEAN SEARCH REPORT**

Application Number

EP 18 15 8483

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | PAIVA JOANA S ET AL: "Changes in ST, QT and RR ECG intervals during acute stress in firefighters: A pilot study", 2016 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 3378-3381, XP032979897, DOI: 10.1109/EMBC.2016.7591452 [retrieved on 2016-10-13] * page 3378 - page 3381 * | 1-15 | INV. A61B5/16 A61B5/0472 A61B5/00 G16H50/20 A61B5/04 ADD. A61B5/024 A61B5/0452 |
| A | MARCO LAURINO ET AL: "Comparative study of morphological ECG features classificators: An application on athletes undergone to acute physical stress", INTELLIGENT SYSTEMS DESIGN AND APPLICATIONS (ISDA), 2011 11TH INTERNATIONAL CONFERENCE ON, IEEE, 22 November 2011 (2011-11-22), pages 242-246, XP032086061, DOI: 10.1109/ISDA.2011.6121662 ISBN: 978-1-4577-1676-8 * the whole document * | 1-15 | |
| A | KESHAN N ET AL: "Machine learning for stress detection from ECG signals in automobile drivers", 2015 IEEE INTERNATIONAL CONFERENCE ON BIG DATA (BIG DATA), IEEE, 29 October 2015 (2015-10-29), pages 2661-2669, XP032837278, DOI: 10.1109/BIGDATA.2015.7364066 [retrieved on 2015-12-22] * the whole document * | 1-15 | TECHNICAL FIELDS SEARCHED (IPC) A61B G16H |
| A | US 2015/257668 A1 (BRAOJOS LOPEZ RUBEN [CH] ET AL) 17 September 2015 (2015-09-17) * the whole document * | 1-15 | |

The present search report has been drawn up for all claims

| Place of search | Date of completion of the search | Examiner |
|---|---|---|
| The Hague | 11 July 2018 | Faymann, Juan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 15 8483

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2018

| Patent document cited in search report | Publication date | Patent family member(s) | Publication date |
|---|---|---|---|
| US 2015257668 A1 | 17-09-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. CUNHA.** PHealth and wearable technologies: A permanent challenge. *Stud. Health Technol. Inform,* 2012, vol. 177, 185-195
- Changes in ST, QT and RR ECG intervals during acute stress in firefighters: A pilot study. **J. PAIVA ; S. RODRIGUES ; J. CUNHA.** Engineering in Medicine and Biology Society (EMBC), 2016 IEEE 38th Annual International Conference of the. IEEE. 2016, 3378-3381
- **A. VOSS ; R. SCHROEDER ; A. HEITMANN ; A. PETERS ; S. PERZ.** Shortterm heart rate variability - influence of gender and age in healthy subjects. *PloS one,* 2015, vol. 10 (3), e0118308
- **M. BIRKETT.** The Trier Social Stress Test protocol for inducing psychological stress. *Journal of visualized experiments: JoVE,* 2011, vol. 19 (56
- **J. CUNHA ; B. CUNHA ; A. PEREIRA et al.** Vital-jacket: A wearable wireless vital signs monitor for patients' mobility in cardiology and sports. *Pervasive Computing Technologies for Healthcare, 2010 4th International Conference on. IEEE,* 2010, 1-2
- Predicting lapses in attention: a study of brain oscillations, neural synchrony and eye measures. **J. PAIVA.** MSc Thesis. University of Coimbra, 2014, 33-36

- **J. CUNHA ; J. PAIVA.** *Biometric Method and Device for Identifying a Person Through an Electrocardiogram (ECG) Waveform - ref. PT109357,* 2016
- **J. PAN ; W. TOMPKINS.** A real-time QRS detection algorithm. *Biomedical Engineering, IEEE Transactions on,* 1985, vol. 32 (3), 230-236
- **D. CLIFFORD.** ECG statistics, noise, artifacts, and missing data. *Advanced Methods and Tools for ECG Data Analysis,* 2006, vol. 6, 55-99
- **H. BAZETT.** An analysis of the time-relations of electrocardiograms. *Heart,* 1920, vol. 7, 353-370
- **KOROS A., D.R.P.S. ; PANJWANI G. ; INGURGIO V. ; D'ARCY J.F.** Complexity in air traffic control towers: A field study part 1. Complexity factors. *Virginia,* 2003, 1-125
- **PAIVA JS ; DIAS D ; CUNHA JPS.** Beat-ID: Towards a computationally low-cost single heartbeat biometric identity check system based on electrocardiogram wave morphology. *PLOS ONE,* 2017, vol. 12 (7), e0180942, https://doi.org/10.1371/journal.pone.0180942